# EUROPÄISCHE PATENTSCHRIFT

(11) **EP 0 959 175 B1**
(45) Veröffentlichungstag und Bekanntmachung des Hinweises auf die Patenterteilung: **17.09.2003**
(21) Anmeldenummer: 99109734.6
(22) Anmeldetag: 18.05.1999
(51) Int. Cl.: D21C 9/10, D06L 3/10, C07C 381/14

(54) **Formamidinsulfinsäure-Abmischungen**
Compositions comprising formamidinesulfinic acid
Compositions comprenant de l'acide formamidine sulfinique

(30) Priorität: 20.05.1998 DE 19822597; 25.07.1998 DE 19833629; 18.11.1998 DE 19853122
(43) Veröffentlichungstag der Anmeldung: 24.11.1999
(73) Patentinhaber: Degussa AG, 40474 Düsseldorf (DE)
(72) Erfinder: Jakob, Harald, Dr., 63594 Hasselroth (DE); Hopf, Bernd, 63791 Karlstein (DE)

(56) Entgegenhaltungen:
- EP-A- 0 824 145
- DE-A- 4 303 320
- US-A- 4 197 256
- US-A- 4 244 780
- DATABASE WPI Section Ch, Week 9226 Derwent Publications Ltd., London, GB; Class E16, AN 92-214279 XP002110820 & JP 04 145064 A (TOKAI ELECTRIC IND CO LTD), 19. Mai 1992 (1992-05-19)
- DATABASE WPI Section Ch, Week 8921 Derwent Publications Ltd., London, GB; Class D25, AN 89-155167 XP002110821 & JP 01 096298 A (LION CORP), 14. April 1989 (1989-04-14)
- DATABASE WPI Section Ch, Week 9442 Derwent Publications Ltd., London, GB; Class A60, AN 94-338256 XP002110822 & JP 06 263721 A (TOKAI DENKA KOGYO KK), 20. September 1994 (1994-09-20)

## Beschreibung

Die Erfindung betrifft Abmischungen die Formamidinsulfinsäure (FAS), mindestens einen in wasser löslichen Komplexbildner und feinteilige Kieselsäure enthalten.

Formamidinsulfinsäure (FAS) wird zur reduktiven Bleiche in der Papier- und Textilindustrie in Form einer wäßrigen, alkalischen Lösung eingesetzt. Während FAS in Wasser relativ schlecht löslich ist (ca. 27 g/l), gehen unter alkalischen Bedingungen bis zu 100 g/l FAS als Sulfinat in Lösung. Für die Bleichflotte liegt das optimale Mengenverhältnis zwischen FAS und Lauge (NaOH, Na₂CO₃) bei etwa 2 : 1. Von Nachteil ist, daß sich die Salze der Formamidinsulfinsäure in wäßriger Lösung rasch zersetzen. Alkalische Bleichlösungen werden daher in der Papierindustrie erst kurz vor dem Zudosieren hergestellt und müssen dann möglichst umgehend verbraucht werden.

Für die kontinuierliche Herstellung und Dosierung von Bleichlösungen aus FAS und NaOH wurde eine spezielle Dosieranlage konzipiert, die bei der Mehrzahl der FAS-Kunden in Betrieb ist. Die Anlagen laufen dabei 24 h/Tag (je nach Bedarf kampagnenweise oder mehrere Monate durchgehend). Manpower zur ständigen Überwachung der FAS-Dosierung steht dabei nicht oder nur wenig zur Verfügung. Während die Zugabe der alkalischen FAS-Bleichlösung bei der kontinuierlich arbeitenden Dosieranlage gut funktioniert, bereitet die FAS-Dosierung immer wieder betriebliche Probleme: FAS backt im Einfülltrichter (Schacht) zusammen und rutscht nicht nach, was eine verminderte Zugabe sowie eine Verdichtung von FAS im Schneckenbereich zur Folge hat. Für Abhilfe sollen derzeit der Einbau von Vibratoren bzw. pneumatischen Klopfern im Schacht sorgen . Durch die schwankende Rieselfähigkeit/Kornbeschaffenheit kommt es oftmals zur schwankenden Dosiermengen bei konstanter Schneckendrehzahl.

Auch die Lagerung von FAS bereitet öfters Schwierigkeiten, da FAS nur eine geringe thermische Stabilität besitzt. So liegt die SADT (self accelerating decomposition temperature) bei 50 ° C. Da je nach Lagerbedingungen immer wieder mit Temperaturen deutlich über 30 Grad zu rechnen ist, kommt es hier zu Problemen mit der Lagerstabilität. Bereits nach wenigen Monaten tritt ein deutliches Nachlassen der Rieselfähigkeit bis hin zum Verklumpen (FAS ist schwach hygroskopisch),eine Gelbfärbung sowie die Erhöhung des Thioharnstoffgehaltes ein. Letzteres ist von besonderer Bedeutung, wenn die in vielen Ländern kennzeichnungspflichtige Grenze von 0,1 % Thioharnstoff (TH) überschritten wird.

Aus der JP 04 145064A (Derwent AN 92-214 279, XP 0021100820) ist eine Abmischung von Thioharnstoffdioxid und feinteiliger Kieselsäure bekannt, bei der die Gelbfärbung bei längerer Lagerung vermieden wird.

Viele in der Industrie gängige Phlegmatisierungsmittel verbessern zwar die thermische Stabilität von FAS, sie können jedoch nicht zugemischt werden, da sie die Bildung eines unangenehmen Schwefelwasserstoff-ähnlichen Geruches bewirken.
Neben der schlechten Rieselfähigkeit stellen insbesondere Kalkablagerungen in Auflösekesseln und Rohrleitungen von FAS-Dosierleitungen ein Problem dar. Bedingt durch den hohen pH-Wert der FAS-Bleichlösungen zeigen gängige Scale-Inhibitoren im allgemeinen eine eingeschränkte Wirkung.

Zur Beseitigung der Kalkablagerungen müssen die Anlagen abgestellt, mit Mineralsäuren gespült und gereinigt werden.

Aufgabe der Erfindung ist es, ein FAS bereitzustellen, das auch bei Lagerung rieselfähig bleibt und bei seiner Anwendung die Bildung von Kalkablagerungen verhindert. Gleichzeitig ist es ein Ziel, die Lagerstabilität durch Phlegmatisierung des FAS zu erhöhen.

Gegenstand der Erfindung sind Abmischungen, die aus FAS, mindestens einem in Wasser löslichen Komplexbildner (pulverförmig oder granuliert) mit einem bevorzugt sauren oder neutralen pH-Wert, und einer feinteiligen, bevorzugt gefällten hydrophilen Kieselsäure bestehen.

Es ist jedoch auch möglich, hydrophobierte Kieselsäuren einzusetzen.
Die Phlegmatisierung des FAS tritt bereits ein, wenn allein die Kieselsäure eingemischt worden ist. Enthält die Abmischung den Komplexbildner und Kieselsäure gemäß der Erfindung, setzt man dem FAS vorteilhaft eine vorher hergestellte Mischung aus Komplexbildner und Kieselsäure in den gewünschten Konzentrationsverhältnissen zu.

Die Kieselsäure wird im allgemeinen in einer Menge von 0,001 Gew.% bis 5 Gew.%, insbesondere 0,01 Gew.% bis 2 Gew.%, bezogen auf die Gesamtmenge der Abmischung zugesetzt.

Die eingesetzten Kieselsäuren weisen im allgemeinen eine BET-Oberfläche (N₂) (DIN 66131) von 60 bis 700 m²/g auf, besonders mit einer Oberfläche von 100 bis 450 m²/g.

Zu den Komplexbildnern zählen in Wasser lösliche Komplexbildner wie Poly-(carboxylate), Polyphosphonsäuren, Zitronensäure, insbesondere aber Polyacrylsäuren, die durch Homopolymerisation erhalten werden, und deren Alkali-, insbesondere Natriumsalze sowie Maleinsäure/Acrylsäure-Copolymerisate und deren Natriumsalze oder deren Gemische. Sie werden in einer Menge von 0,05 bis 4 Gew.%, bevorzugt 0,1 bis 2 Gew.%, bezogen auf die Gesamtmenge der Abmischung, in dieser in fester Form eingesetzt.

Gegenstand der Erfindung ist auch die Verwendung von Polyacrylsäuren, in denen als Copolymerisat ca. 10 % Polyamid enthalten sind.

Zu den eingesetzten Polycarboxylaten gehören insbesondere solche der allgemeinen Struktur (X, Y), in denen X für 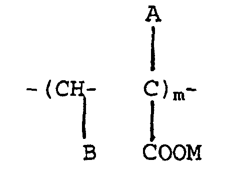 und Y für

- (F)_{q}-

steht, mit den Bedeutungen für:
- A =: H, OH, C₁-₆ Alkyl, CH₂COOM;
- B =: H, OH, C₁-₆ Alkyl, COOM;
- F =: ein copolymerisierbares Monomer;
- M =: H, Alkali- bzw. Erdalkalimetall, Ammonium, substituiertes Ammonium
und
- m =: 0,5-100 Mol%
- q =: 0 - 99,5 Mol %.

Diese Verbindungen sind bekannt aus der DE-A-4303320 mit der Verwendung als Cobuilder in Waschmitteln.

Die Polycarboxylate können sowohl in Form von Säuren als auch als Salze bzw. als teilneutralisierte Substanzen verwendet werden; als Gegenionen eignen sich Metallionen sowie stickstoffhaltige Kationen.

Die erfindungsgemäß eingesetzten Polymerisate der Strukturen (X, Y) sind bevorzugt Homo- oder Copolymere der Acrylsäure. Die Verteilung der Monomeren im Polymer ist üblicherweise statistisch (random). Das copolymerisierbare Monomer F wird vorteilhaft so gewählt, daß es die belagsverhindernde Wirkung des gesamten Polymer nicht beeinträchtigt. Geeignete Monomere F sind monoethylenisch ungesättigte, carboxylgruppenfreie Monomere, wie z. B. Hydroxy(meth)acrylate mit (CH₂)ₓOH als Estergruppe, wobei x = 2-4 ist. (Meth)acrylamid, (Meth)acrylonitril, Vinylsulfonsäure/Allylsulfonsäure, Dimethylaminoethyl (meth) acrylat, Diethylaminoethyl(meth)acrylat, 2-(meth)acrylamido-2-methylpropan-sulfonsäure, Vinylphosphonsäure, Allylphosphonsäure, Allylalkohol, Vinylglykol, Vinylacetat, Allylacetat, N-Vinylpirrolidon, N-vinylformamid, N-Vinylimidazol, N-vinylimidazolin,1-Vinyl,-2-methyl-2-imidazolin, Ester der (Meth)acrylsäure mit 1-8 C-Atome im Alkoholrest, wie z. B. Methyl(meth)acrylat, Ethyl (meth) acrylat, Propyl(meth) acrylat), butyl(meth)acrylat evtl. mit Alkohol- bzw. mit Aminogruppen funktionalisiert,Ethylen, Propylen, Methylvinylether, Ethylvinylether, Styrol und alphaMethyistyrol. Alle monomeren Säuren und Basen können evtl. auch als Salze verwendet werden.

Mehrfachethylenisch ungesättigte Monomere sind z. B. Ester aus Ethylenglykol, Propylenglykol, Butandiol bzw. Hexandiol mit (Meth)acryl-, Malein- bzw. Fumarsäure, Ester aus Polyethylglykol bzw. Copolmeren aus Ethylen- und Propylenglykol mit (Meth)acrylsäure, Maleinsäure bzw. Fumarsäure, zwei- bis dreifach mit (Meth)acryl- bzw. Maleinsäure veresterte Anlagerungsprodukte von Ethylenoxid und/oder Propylenoxid an Trimethylolpropan, mindestens zweifache Ester aus (Meth)acryl- bzw. Maleinsäure und Glycerin bzw. Pentaerythrit, Triallylamin, Tetraallylethylendiamin, Polyethylenglykoldivinylether, Trimethylolpropandiallylether, Butandioldiallylether, Pentaerythrittriallylether, Divinylharnstoff.

Für die Komponente X eignen sich monoethylenisch 5 ungesättigte C₃ bis C₈ Mono- bzw. Dicarbonsäuren, wie z. B. Acrylsäure, Methacrylsäure, Fumarsäure, Maleinsäure sowie Maleinsäureanhydrid, Itaconsäure, Citraconsäure, Crotonsäure.Auch geeignet sind die aus diesen Verbindungen abgeleiteten Ester sowie Amide.

Ebenfalls geeignet sind aus diesen Verbindungen abgeleitete Polymere, die sich aus verschiedenen monomeren Bausteinen des Strukturelementes X zusammensetzen.

Die Differenz zu dem (den) einzeln oder gemeinsam vorliegenden Komplexbildner der Kieselsäure wird auf 100 Gew.-% mit handelsüblichem FAS aufgefüllt.

Es ist auch möglich, Stickstoff-haltige Carbonsäuren, wie z. B. EDTA oder DTPA einzusetzen. Dabei ist unter den in der Bleichflotte herrschenden pH-Werten mit einer eingeschränkten Wirksamkeit zu rechnen.

Die Abmischungen werden auf bekanntem Weg hergestellt und lassen sich unter Praxisbedingungen gut auflösen. Der Bleicheffekt ändert sich gegenüber dem die erfindungsgemäßen Beimischungen nicht enthaltenden FAS nicht.

Versuche mit FAS-Abmischungen, die 0,3 Gew.% eines Gemisches aus einer handelsüblichen sprühgetrockneten Kieselsäure und einer Polyacrylsäure enthalten, führen zu einem auch nach mehreren Monaten noch rieselfähigen Produkt, das auch die angestrebte Lagerstabilität aufweist.

Beläge bildeten sich unter den für die Bewertung ausschlaggebenden Betriebsbedingungen im Auflösekessel oder den Rohrleitungen nicht.

## Patentansprüche

1. Abmischungen, bestehend, aus Formamidinsulfinsäure (FAS), mindestens einem in Wasser löslichen Komplexbildner feinteiliger Kieselsäure.

2. Abmischungen gemäß Anspruch 1,
**dadurch gekennzeichnet,**
daβ sie 0,05 bis 4 Gew.% des/der in Wasser löslichen Komplexbildner(s) und 0,001 bis 5 Gew.% der Kieselsäure enthalten, jeweils bezogen auf die Gesamtmenge der Abmischung.

3. Abmischung gemäß den Ansprüchen 1 oder 2,
**dadurch gekennzeichnet**
**daß** der/die in Wasser lösliche Komplexbildner einen sauren oder neutralen pH-Wert aufweist (aufweisen).

4. Abmischungen gemäß einem oder mehreren der vorhergehenden Ansprüche,
**dadurch, gekennzeichnet,**
**daß** der/die in Wasser lösliche Komplexbildner einzeln oder im Gemisch ein Poly-(carboxylat), eine Polyphosphonsäure oder Zitronensäure oder deren lösliches Salz, insbesondere Alkalisalz ist (sind).

5. Abmischungen gemäß den Ansprüchen 1 bis 3,
**dadurch gekennzeichnet,**
**daß** der/die in Wasser lösliche Komplexbildner eine Polyacrylsäure oder deren Natriumsalz ist (sind).

6. Abmischungen gemäß den Ansprüchen 1 bis 3,
**dadurch gekennzeichnet,**
**daß** der/die in Wasser lösliche Komplexbildner ein Maleinsäure/Acrylsäure-Copolymerisat oder dessen Natriumsalz ist (sind).

7. Abmischungen gemäß Anspruch 5,
**dadurch gekennzeichnet,**
**daß** die eingesetzte Polyacrylsäure ca. 10 % eines Polyamids in Form eines Copolymerisats enthält.

8. Abmischungen gemäß Anspruch 4,
**dadurch gekennzeichnet,**
**daß** der/die in Wasser lösliche Komplexbildner einem Polycarboxylat der allgemeinen Struktur (X, Y) entspricht in der X für 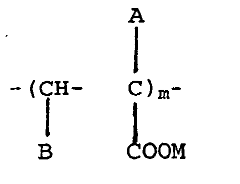 und Y für
-(F)_{q}-
steht, mit den Bedeutungen für:
A = H, OH, C₁-₆ Alkyl, CH₂COOM;
B = H, OH, C₁-₆ Alkyl, COOM;
F = ein copolymerisierbares Monomer;
M = H, Alkali- bzw. Erdalkalimetall, Ammonium, substituiertes Ammonium;
und
m = 0,5-100 Mol%
q = 0 - 99,5 Mol %.

9. Abmischungen gemäß Anspruch 7,
**dadurch gekennzeichnet,**
**daß** der in Wasser lösliche Komplexbildner eimen Polycarboxylat entspricht, der sich aus Bausteinen des Strukturelements X zusammensetzt.

10. Verwendung der Abmischungen gemäß einem oder mehreren der vorhergehenden Ansprüche zur reduktiven Bleiche.

## Claims

1. Blends consisting of formamidinesulfinic acid (FSA), at least one water-soluble complexing agent and finely divided silicic acid.

2. Blends according to Claim 1,
**characterised in that**
they contain 0.05 wt.% to 4 wt.% of the water-soluble complexing agent(s) and 0.001 wt.% to 5 wt.% of the silicic acid, in each instance relative to the total quantity of the blend.

3. Blends according to Claims 1 or 2,
**characterised in that**
the water-soluble complexing agent(s) has/have an acidic or neutral pH value.

4. Blends according to one or more of the preceding claims,
**characterised in that**
the water-soluble complexing agent(s), individually or in a mixture, is/are a poly(carboxylate), a polyphosphonic acid or citric acid or a soluble salt thereof, in particular an alkali salt.

5. Blends according to Claims 1 to 3,
**characterised in that**
the water-soluble complexing agent(s) is/are a polyacrylic acid or a sodium salt thereof.

6. Blends according to Claims 1 to 3,
**characterised in that**
the water-soluble complexing agent(s) is/are a maleic-acid/acrylic-acid copolymer or a sodium salt thereof.

7. Blends according to Claim 5,
**characterised in that**
the polyacrylic acid employed contains about 10 % of a polyamide in the form of a copolymer.

8. Blends according to Claim 4,
**characterised in that**
the water-soluble complexing agent(s) corresponds to a polycarboxylate of the general structure (X, Y), in which X stands for 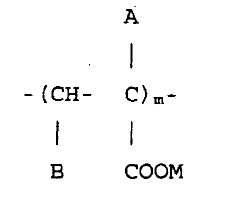 and Y stands for
- (F)_{q}-
with the following meanings:
A = H, OH, C₁₋₆ alkyl, CH₂COOM;
B = H, OH, C₁₋₆ alkyl, COOM;
F = a copolymerisable monomer;
M = H, alkali metal or alkaline-earth metal, ammonium, substituted ammonium;
and
m = 0.5-100 mol %
q = 0 - 99.5 mol %.

9. Blends according to Claim 7,
**characterised in that**
the water-soluble complexing agent corresponds to a polycarboxylate which is composed of building blocks representing structural element X.

10. Use of the blends according to one or more of the preceding claims for the purpose of reductive bleaching.

## Revendications

1. Compositions constituées d'acide formamidine sulfinique (AFS), d'au moins un agent complexant soluble dans l'eau et d'acide silicique finement divisé.

2. Compositions selon la revendication 1,
**caractérisées en ce que**
elles contiennent 0,05 à 4 % en poids du (des) agent(s) complexant(s) soluble(s) dans l'eau et 0,001 à 5 % en poids de l'acide silicique, par rapport à chaque fois à la quantité totale de la composition.

3. Compositions selon la revendication 1 ou 2,
**caractérisées en ce que**
le (les) agent(s) complexant(s) soluble(s) dans l'eau a(ont) un pH acide ou neutre.

4. Compositions selon l'une ou plusieurs des revendications précédentes,
**caractérisés en ce que**
le(les) agent(s) complexant(s) soluble(s) dans l'eau est(sont), individuellement ou en mélange, un poly(carboxylate), un acide polyphosphonique ou l'acide citrique ou leur sel soluble, en particulier un sel alcalin.

5. Compositions selon les revendications 1 à 3,
**caractérisées en ce que**
le(les) agent(s) complexant(s) soluble(s) dans l'eau est (sont) un acide polyacrylique ou son sel de sodium.

6. Compositions selon les revendications 1 à 3,
**caractérisées en ce que**
le(les) agent(s) complexant(s) soluble(s) dans l'eau est (sont) un copolymère acide maléïque / acide acrylique ou son sel de sodium.

7. Compositions selon la revendication 5,
**caractérisées en ce que**
l'acide polyacrylique utilisé contient environ 10 % d'un polyamide sous forme d'un copolymère.

8. Compositions selon la revendication 4,
**caractérisées en ce que**
à l'agent (aux agents) complexant(s) soluble(s) dans l'eau, correspond un polycarboxylate de structure générale (X,Y) dans laquelle X est 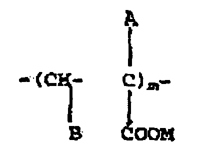 et Y est-(F)q-
avec les significations :
A = H, OH, alkyle en C₁₋₆, CH₂COOM;
B = H, OH, alkyle en C₁₋₆, COOM ;
F = un monomère copolymérisable;
M = H, un métal alcalin ou alcalino-terreux, ammonium, ammonium substitué ;
et
m = 0,5 - 100 mole(s) %
q = 0 - 99,5 mole(s) %.

9. Compositions selon la revendication 7,
**caractérisées en ce que**
l'agent complexant soluble dans l'eau correspond à un polycarboxylate, qui est composé d'éléments constitutifs de l'élément de structure X.

10. Utilisation des mélanges selon l'une ou plusieurs des revendications précédentes pour blanchiment réducteur.
